# NEW EUROPEAN PATENT SPECIFICATION

(11) **EP 2 028 937 B2**
(45) Date of publication and mention of the opposition decision: **27.06.2018**
(45) Mention of the grant of the patent: 28.01.2015
(21) Application number: 07797634.8
(22) Date of filing: 22.05.2007
(51) Int. Cl.: A61K 31/405, A61P 43/00

(54) **MELATONIN AGONIST TREATMENT**
BEHANDLUNG MIT MELATONINAGONISTEN
TRAITEMENT PAR UN AGONISTE DE LA MÉLATONINE

(30) Priority: 22.05.2006 US 747847 P
(43) Date of publication of application: 04.03.2009
(73) Proprietor: Vanda Pharmaceuticals Inc., Washington, DC 20037 (US)
(72) Inventor: BIRZNIEKS, Gunther, Bethesda, MD 20817 (US); PHADKE, Deepak, Olathe, KS 66062 (US); POLYMEROPOULOS, Mihael, H., Potomac, MD 20854 (US)
(74) Representative: Carpmaels & Ransford LLP
(86) International application number: PCT/US2007/069411
(87) International publication number: WO 2007/137244

(56) References cited:
- WO-A1-2007/016203
- WO-A1-2016/066631
- WO-A2-2005/063297
- US-A- 5 420 152
- US-A- 5 856 529
- US-A1- 2009 105 333
- RAJARATNAM S M ET AL: "The melatonin agonist VEC-162 immediately phase-advances the human circadian system" SLEEP, ALLEN PRESS, LAWRENCE, KS, US, vol. 29, no. Suppl. S, 26 June 2006 (2006-06-26), XP008098612 ISSN: 0161-8105
- NIMISH N VACHHARAJANI ET AL: "PRECLINICAL PHARMACOKINETICS AND METABOLISM OF BMS-214778, A NOVEL MELATONIN RECEPTOR AGONIST" JOURNAL OF PHARMACEUTICAL SCIENCES, AMERICAN PHARMACEUTICAL ASSOCIATION, WASHINGTON, US LNKD- DOI:10.1002/JPS.10348, vol. 92, no. 4, 1 April 2003 (2003-04-01), pages 760-772, XP008074135 ISSN: 0022-3549
- KIPPAX P.: 'Systematic method development for laser diffraction particle sizing' AMERICAN LABORATORY August 2004,
- FOLIA PHARMACOL. JPN. vol. 129, 12 January 2007, pages 35 - 41
- DALZIEL C.: 'JPMorgan 24th annual healthcare conference' IDRUGS vol. 9, no. 3, 2006, pages 182 - 184
- ERMAN M. ET AL: 'An efficacy, safety, and dose-response study of ramelteon in patients with chronic primary insomnia' SLEEPMEDICINE vol. 7, 2006, pages 17 - 24
- Lockley S.W. et al, www.thelancet.com Published online 5 August 2015, http://dx.doi.org/10.1016/50140-6736(15)600 31-9
- HUNT A.E. ET AL: 'Activation of MT2 melatonin receptors in rat suprachiasmatic nucleus phase advances the circadian clock' AM. J. PHYSIOL CELL PHYSIOL vol. 280, 2001, pages C110 - C118
- ZEE P.C. ET AL: 'Effects of ramelteon on insomnia symptoms induced by rapid, eastward travel' SLEEP MEDICINE vol. 11, 2010, pages 525 - 533
- ARENDT J.: 'Melatonin and human rhythms' CHRONOBIOLOGY INTERNATIONAL vol. 23, no. 1&2, 2006, pages 21 - 37
- SPITZER R.L. ET AL: 'Jet lag: Clinical features, validation of a new syndrome-specific scale, and lack of response to melatonin in a randomized, double-blind trial' AM. J. PSYCHIATRY vol. 156, no. 9, September 1999, pages 1392 - 1396
- BUSCEMI N. ET AL: 'Melatonin for treatment of sleep disorders; Summary, Evidence Report/ Technology Assessment' AHRQ AGENCY FOR HEALTHCARE RESEARCH AND QUALITY no. 108, November 2004, pages 1 - 8
- LAVEDAN C. ET AL: 'Tasimelteon: A selective and unique receptor binding profile' NEUROPHARMACOLOGY vol. 91, 2015, pages 142 - 147
- LAUDON M. ET AL: 'Therapeutic effects of melatonin receptor agonists on sleep and comorbid disorders' INT. J. MOL. SCI. vol. 15, 2014, pages 15924 - 15950
- KIPPAX P.: 'Issues in the appraisal of laser diffraction; Particle Sizing Techniques' PHARMACEUTICAL TECHNOLOGY EUROPE 2005, pages 32 - 39
- DIJK D-J. ET AL: 'Contribution of the circadian pacemaker and the sleep homeostat to sleep propensity, sleep structure, electroencephalographic slow waves, and sleep spindle activity in humans' THE JOURNAL OF NEUROSCIENCE vol. 15, no. 5, May 1995, pages 3526 - 3538
- CROWLEY S.J. ET AL: 'Melatonin in the afternoons of a gradually advancing sleep schedule enhances the circadian rhythm phase advance' PSYCHOPHARMACOLOGY (BERL) vol. 225, no. 4, February 2013, pages 825 - 837

## Description

### BACKGROUND OF THE INVENTION

### Field of the Invention

This invention is in the field of melatonin agonists for pharmaceutical uses.

### Related Art

The compound referred to herein as MA-1 is (1R-trans)-N-[[2-(2,3-dihydro-4-benzofuranyl)cyclopropyl]methyl] propanamide. It is disclosed in U.S. 5,856,529.

MA-1 is a specific and potent agonist of the MT1 R and MT2R melatonin receptors in the Suprachiasmatic nucleus (SCN), the region of the brain associated with the biological clock. (Kokkola,T. & Laitinen,J.T. Melatonin receptor genes. Ann. Med 30, 88-94 (1998).) Engagement of these receptors by melatonin is believed to regulate circadian rhythms, including the sleep/wake cycle. Consistent with its receptor binding profile, MA-1 demonstrates potent chronobiotic activity in preclinical models of acute phase-shifting and chronic re-entrainment.

Previous studies showed that MA-1 is well-tolerated by healthy volunteers in single doses up to 300 mg and in multiple doses (up to 28 days) up to 150 mg. A 28-day Phase II study was also conducted to investigate the effects of MA-1 in elderly patients with primary insomnia. In this study MA-1 did not differentiate from placebo with respect to sleep latency and the number of nocturnal awakenings. While patients with the lowest melatonin levels may have benefited from MA-1 treatment more than placebo, the design of this study made it difficult to interpret the effects of MA-1 on the sleep-wake cycle.

WO 2007/016203 A1 discloses various imidazolylalkyl-pyridines that are used as wakefulness compounds.

US 5 856 529 A discloses the use of melatonin agonists for treating sleep disorders and other chronobiological disorders such as jet lag, work shift syndrome etc.

### SUMMARY OF THE INVENTION

This invention relates to the discovery of effective doses of MA-1 in the treatment of sleep disorders and circadian rythm disorders.

### DETAILED DESCRIPTION

This invention, which is hereinafter described with respect to illustrative embodiments, contemplates the melatonin agonist herein referred to as MA-1 for use in the treatment of sleep disorders and circadian rhythm disorders. MA-1 is a white to off-white powder with a melting point of about 78°C (DSC) and has the structure illustrated in Formula 1.

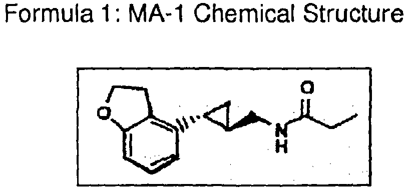

MA-1 may be internally administered to a patient, typically an adult, of typical size, e.g., approximately 70 kg and typically within the range of about 45 to about 150 kg, who is in need thereof in doses of about 20 mg/day or about 50 mg/day.

Typically the drug may be administered in immediate release form but controlled release forms can also be used. The drug can be delivered alone or in combination with another active pharmaceutical ingredient.

The route of administration is usually oral although other routes of administration, e.g., parenteral, intravenous, intramuscular, buccal, lozenge, transdermal, transmucosal, etc., can be used. Controlled release forms, e.g., sustained, pulsatile, or delayed, including depot forms such as are disclosed in WO2003037337 or WO2004006886, can also be used.

The compositions are formulated in an oral unit dosage form, each dosage containing from about 20 to about 50 mg of MA-1. The term "unit dosage form" refers to physically discrete units suitable as unitary dosages for human subjects, each unit containing a predetermined quantity of active material calculated to produce the desired prophylactic or therapeutic effect over the course of a treatment period, in association with the required pharmaceutical carrier. So, for example, an adult patient suffering a circadian rhythm disorder could be prescribed 1-4 tablets, each having about 5 to about 50 mg of MA-1 for a total daily dose of about 20 or about 50 mg/day. The term, "about" means, in general, a range of plus or minus ten percent, except that with respect to whole single digit or fractional values, the range is within plus or minus one of the last digit recited. Thus, "about 100" includes 90 to 110, "about 5" includes 4 to 6, and "about 1.5" includes 1.4 to 1.6. In no event can the term, "about," include a nonsensical value such as a value that exceeds 100% or is less than zero.

An effective amount, quantitatively, may vary, e.g., depending upon the patient, the severity of the disorder or symptom being treated, and the route of administration. Such dose can be determined by routine studies. In general, for systemic administration, e.g., oral administration, the dose of MA-1 will be about 20 or about 50 mg/day, in one or more unit dosage forms.

It will be understood that the dosing protocol including the amount of MA-1 actually administered will be determined by a physician in the light of the relevant circumstances including, for example, the condition to be treated, the chosen route of administration, the age, weight, and response of the individual patient, and the severity of the patient's symptoms. Patients should of course be monitored for possible adverse events.

Particle size will also affect the dose selected. At larger particle sizes, i.e., D50 is greater than about 100 um, e.g., about 100 to about 200 um, oral doses at the higher end, i.e., up to about 100 mg are effective, whereas at smaller particle sizes, i.e., D50 is less than about 100 um, e.g., about 20 to about 50 um, lower doses, i.e., less than about 100 mg, are useful, e.g., about 10 mg to about 80 mg and about 20 mg to about 50 mg. (Particle size measurements supporting the above were made laser diffraction using a Malvern Mastersizer. The D50 (D10, D90, D100) value means that 50% (10%, 90%, 100%) of the particles by weight are of the indicated diameter or smaller.) The above doses may be administered in immediate release form, i.e., a non-controlled release formulation.

If desired, doses can optionally be adjusted for body size using the following as guidance: useful amounts for larger particles are up to about 1.5 mg/kg; useful amounts for smaller particles include doses of less than about 1.5 mg/kg, e.g., about .1 mg/kg to about 1.2 mg/kg and about .3 mg/kg to about .7 mg/kg.

Treatment is continued until the patient's circadian rhythm is restored to normal, i.e., until the patient's normal daily functioning is not inhibited by the circadian rhythm disorder or, in the case of a sleep disorder, until the patient is sleeping normally, i.e., until the patient's normal daily functioning is not inhibited by the sleep disorder. Treatment can continue for some time after these end points are achieved so as to lessen the likelihood of relapse.

For therapeutic or prophylactic use, MA-1 may normally be administered as a pharmaceutical composition comprising as the (or an) essential active ingredient at least one such compound in association with a solid or liquid pharmaceutically acceptable carrier and, optionally, with pharmaceutically acceptable adjuvants and excipients employing standard and conventional techniques.

MA-1 is very soluble or freely soluble in 95% ethanol, methanol, acetonitrile, ethyl acetate, isopropanol, polyethylene glycols (PEG-300 and PEG-400), and only slightly soluble in water. The native pH of a saturated solution of MA-1 in water is 8.5 and its aqueous solubility is practically unaffected by pH.

Pharmaceutical compositions may include suitable dosage forms for oral, parenteral (including subcutaneous, intramuscular, intradermal and intravenous), transdermal, bronchial or nasal administration. Thus, if a solid carrier is used, the preparation may be tableted, placed in a hard gelatin capsule in powder or pellet form, or in the form of a troche or lozenge. The solid carrier may contain conventional excipients such as binding agents, fillers, tableting lubricants, disintegrants, wetting agents and the like. The tablet may, if desired, be film coated by conventional techniques. If a liquid carrier is employed, the preparation may be in the form of a syrup, emulsion, soft gelatin capsule, sterile vehicle for injection, an aqueous or non-aqueous liquid suspension, or may be a dry product for reconstitution with water or other suitable vehicle before use. Liquid preparations may contain conventional additives such as suspending agents, emulsifying agents, wetting agents, non-aqueous vehicle (including edible oils), preservatives, as well as flavoring and/or coloring agents. For parenteral administration, a vehicle normally will comprise sterile water, at least in large part, although saline solutions, glucose solutions and like may be utilized. Injectable suspensions also may be used, in which case conventional suspending agents may be employed. Conventional preservatives, buffering agents and the like also may be added to the parenteral dosage forms. Particularly useful is the administration of a compound of Formula I in oral dosage formulations. The pharmaceutical compositions may be prepared by conventional techniques appropriate to the desired preparation containing appropriate amounts of MA-1. *See*, for example, Remington's Pharmaceutical Sciences, Mack Publishing Company, Easton, Pa., 17th edition, 1985.

In making pharmaceutical compositions, the active ingredient(s) will usually be mixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, excipient, or medium for the active ingredient. Thus, the composition can be in the form of tablets, pills, powders, lozenges, sachets, cachets, elixirs, suspensions, emulsions, solutions, syrups, aerosols (as a solid or in a liquid medium), ointments containing for example up to 10% by weight of the active compound, soft and hard gelatin capsules, suppositories, sterile injectable solutions and sterile packaged powders.

Some examples of suitable carriers and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, methyl cellulose, methyl- and propylhydroxybenzoates, talc, magnesium stearate and mineral oil. The formulations can additionally include lubricating agents, wetting agents, emulsifying and suspending agents, preserving agents, sweetening agents or flavoring agents. The compositions may be formulated so as to provide quick, sustained or delayed release of the active ingredient after administration to the patient.

The compositions are preferably formulated in a unit dosage form, each dosage containing from about 20 to about 50 mg of the active ingredient. The term "unit dosage form" refers to physically discrete units suitable as unitary dosages for human subjects and other mammals, each unit containing a predetermined quantity of active material calculated to produce the desired prophylactic or therapeutic effect over the course of a treatment period, in association with the required pharmaceutical carrier. So, for example, an adult patient suffering a depressive disorder could be prescribed 1-4 tablets, each having 5-50 mg of MA-1, to be taken once, twice or three times daily and might expect improvement in his or her condition within about one to about 12 weeks.

A typical unit dose form could be size 0 or size 1 capsule comprising 20 or 50 mg of MA-1 in addition to anhydrous lactose, microcrystalline cellulose, silicon dioxide colloidal, croscarmellose sodium, and magnesium stearate. Storage at 15 to 20 °C with protection from moisture and sunlight is recommended.

The D50 of the MA-1 administered may be less than about 100 um, for example, about 20 to about 50 um or about 30 to 40 um.

MA-1 can also be formulated in a controlled release form, e.g., delayed, sustained, or pulsatile release. MA-1 can also be administered concomitantly with other drug therapies, including but not limited to other antidepressant drug therapies or other drug therapies for treating other emotional disorders. So, for example, MA-1 may be administered in combination with other melatonergic agonists or other sleep-inducing agents.

### Examples

### Example 1.

A clinical trial was conducted to assess the safety of MA-1 as well as to determine the ability of MA-1 to shift the sleep/wake cycle following a 5 hour advance in bedtime. The study was a randomized, double-blind, parallel group, placebo-controlled study. It consisted of a 2-4 week outpatient screening period followed by an 8-day inpatient stay. After acclimating to the sleep lab, bedtime was advanced by 5 hours. The primary objectives of this study were to investigate the exposure-response to MA-1 on advancement of circadian release of endogenous melatonin rhythm as measured by dim light melatonin onset (DLMO, a biomarker of the sleep-wake cycle), to investigate the exposure-response to MA-1 on mean sleep efficiency parameters as measured by PSG, to investigate the exposure-response to MA-1 on objective neurobehavioral performance lapses during scheduled work-time as measured by computerized continuous performance testing, and to assess the safety and tolerability of MA-1. Forty-five healthy volunteers, men and women aged 18-50, were enrolled into this study. Thirty-nine subjects were randomized. The results of this study are presented below.

The study was designed to assess the safety and efficacy of four oral doses of MA-1 (10 mg, 20 mg, 50 mg and 100 mg) compared to matching placebo on circadian phase shift, sleep parameters during the major sleep episode, and subject alertness. After written informed consent was signed, subjects that met the inclusion/exclusion criteria at screening and baseline were enrolled into the 8-day in-patient portion of the study. All in-patient assessments were conducted in a time-isolation sleep lab in which no time cues were available to subjects. During the first three nights, subjects were given placebo 30 minutes prior to bedtime (11:00 PM) in a single-blind fashion. Baseline assessments for the efficacy parameters were measured during this period. At 5:00 PM on day 3, subjects started a 19 hour Preconstant posture (CP) segment during which time the subjects remained seated in a semi-recumbent position and blood samples were collected approximately every hour from 7:00 AM to 12:00 PM. The purpose of the pre-CP segment is to provide a measure of each subject's circadian phase before the start of the night shift segment. On day 4, subjects were randomized to once daily treatment in one of the five treatment groups. In addition, subject sleep-wake routines were advanced 5 hours, such that subjects were required to sleep from approximately 6:00 PM - 2:00 AM. Treatment was administered and the time shift was maintained for 3 days. Efficacy parameters were collected during this time. To measure circadian phase at the end of the study, a 24-hour post-CP was conducted immediately after the treatment segment on day 7. Over the course of the study, approximately 500 mL of blood was drawn from each subject. Safety was assessed throughout the study, at the end of study (EOS) visit on day 8, and at the Follow-up visit. The target number of subjects for enrollment was 40 but 45 were actually enrolled.

The particle size of the MA-1 used in this study was:

| D10 | D50 | D90 | D100 |
|---|---|---|---|
| 10 um | 115 um | 316 um | 631 um |

It is hypothesized that in order to achieve maximum efficacy peak plasma concentrations of MA-1 should coincide with the time that subjects go to bed. Since peak plasma concentration (Cₘₐₓ) is reached at 0.5-1 hour after oral administration, MA-1 was administered 30 minutes prior to bedtime. A placebo control was used to distinguish the effects of the drug from other components of treatment in the study population over a defined treatment period.

The oral doses selected were based on safety and efficacy data obtained from previous MA-1 pre-clinical and clinical trials. In vitro pharmacologic models of acute and chronic phase-shifting demonstrated chronobiotic activity at doses ranging from 1 to 5 mg/kg. Extrapolation of these data to humans suggests that the 0.14 to 0.71 mg/kg, or 10 to 50 mg in a 70 kg subject, should effectively advance the sleep-wake cycle. Though not optimally designed to assess the chronobiotic potential of MA-1, clinical trial CN116-002 measured the effects MA-1 on the circadian sleep-wake cycle. Results from that study showed that 50 mg MA-1 consistently shifted circadian rhythms. The doses selected for the study (10, 20, 50 and 100 mg) were within the expected dose range for efficacy. The safety of the selected doses for this study is supported by previous clinical studies. In Phase I clinical trials, a single oral dose of 1 to 300 mg of MA-1 was safe and well tolerated in healthy subjects. Additionally, safety and tolerability of MA-1 at doses up to 150 mg has been demonstrated in daily administration for 28 days in healthy subjects and elderly subjects with chronic insomnia. The highest dose in the study, 100 mg, is well within the safety margin established in both Phase I single and multiple ascending dose trials and in a Phase II study.

To assess circadian sleep-wake cycles in the study, plasma melatonin levels were assessed. The onset of melatonin production, or dim light melatonin onset (DLMO), is associated with onset of sleep. DLMO is considered a standard marker used frequently to assess circadian phase. To assess the effects of MA-1 on the sleep-wake cycles, DLMO was monitored in subjects before and after treatment. For this study, DLMO was defined as the time when melatonin production reaches 25% of the nightly peak (MEL25%up) of the fitted melatonin phase curve.

Because light has a significant confounding effect on melatonin release, light levels in the sleep laboratory were carefully regulated. Subjects were exposed to a light intensity of 25 lux in the angle of gaze (50 lux maximum light intensity in the room) during the awake portions of the protocol, except in the first 6 hours of the CP segment. Twenty-five lux in the angle of gaze was chosen because this low intensity reduces the phase-shifting effect of light and is also consistent with the light exposure many shift workers experience at work. Subjects were exposed to a light intensity of less than 2 lux in the angle of gaze (8 lux maximum intensity in the room) during the first 6 hours of the CP segments. Endogenous melatonin production, including the onset and maximum plasma concentration, is measured during the CP portion of the protocol. Low light intensity was chosen to eliminate the effect of light on endogenous melatonin secretion.

### DLMO

DLMO is a biomarker of the circadian sleep-wake cycle. One of the primary objectives of this study was to investigate the exposure-response of MA-1 on the sleep-wake cycle as measured by DLMO. To construct the melatonin phase curve, plasma melatonin levels (pg/ml) were measured once every 30 minutes during the first 14 hours of the CP segments and hourly for the remainder of the CP segments. The full melatonin phase curve was constructed so that peak melatonin concentrations could be defined. Based on peak melatonin concentrations, DLMO, defined as 25% of the peak, was determined. During double-blind treatment (Days 4-6), plasma melatonin levels were measured every 30 minutes from 4:00 PM to 2:00 AM. This window of time was estimated to contain the DLMO. To determine if any dose of MA-1 induced a phase-shift in circadian rhythm, the difference between DLMO on treatment days and baseline for MA-1-treated subject was compared against the difference between DLMO on treatment days and baseline for placebo-treated subjects.

### Sleep Efficiency

Another primary objective of this study was to investigate the exposure-response to MA-1 on mean sleep efficiency parameters. Sleep efficiency (time asleep/time in bed * 100%) was measured using polysomnography (PSG). A variety of sensors were applied to the subjects with paste or tape through which brain waves, eye movements, muscle tone, body movements, heart rate, and breathing were recorded. Audiovisual recordings were also taken. PSG recording was done during the sleep episodes of days 1, 2, 3, 4, 5, 6, and 7 of this study (referred to as Nights 1-7). Sleep efficiencies of MA-1-treated subjects were compared with sleep efficiencies from placebo-treated subjects. Data from PSG on Nights 3 and 7 were not analyzed.

### Other Polysomnography Parameters

Sleep parameters were recorded during all sleep episodes (11:00 PM to 7:00 AM on Nights 1, 2, and 3, and 6:00 PM to 2:00 AM on Nights 4, 5, 6, and 7). From these recordings sleep latency (latency to persistent sleep) and wake after sleep onset (WASO) were calculated. PSG on Nights 3 and 7 was not analyzed.

### Efficacy Analyses

### Primary Efficacy Variables

### Dim Light Melatonin Onset

Peak melatonin was determined from a subject's melatonin values as the mean of the maximal values obtained on Night 3 and Night 7; if melatonin was not sampled on one of these days (or if there were inadequate samples obtained during the period at which melatonin should peak), peak melatonin was the peak for the other day. For the primary analysis, threshold was calculated as 25% of peak melatonin (DLMO25%). DLMO was calculated by linear interpolation of these melatonin values and the corresponding time points.

The differences in DLMO25% between the endpoint day (Nights 4, 5 and 6) and baseline (Night 3) were analyzed by comparing pairwise each dose group to placebo using a linear one-way analysis of variance (ANOVA) model using in SAS® (SAS® Institute, Cary, North Carolina). Means were calculated using the LS Means method in SAS®. Standard deviations were calculated using the Statistical Summary function in SAS®. Other statistical tests were also presented in graphics. These included: linear regression of response *vs*. exposure (dose, AUC, or Cmax), Kendall-tau nonparametric regression, and Spearman nonparametric regression.

### Sleep Efficiency

Another primary outcome of interest was sleep efficiency (SE). SE (%) was defined as the total time asleep divided by the time allowed as an opportunity for sleep in a period multiplied by 100%. SE over portions of the night was also analyzed, including first and second halves of the night, and first, second and final thirds of the night. Time allowed for sleep was 8 hours (480 minutes).

The effect of treatment (Nights 4, 5, and 6) vs. baseline (Night 2) was based on the difference between SE values on these days. The overall mean sleep efficiency on Nights 4, 5, and 6 was also calculated and compared to baseline. The same baseline and endpoint days were used for the portions of the night analyses. The differences in SE between the endpoint day and baseline were analyzed by comparing pairwise each dose group to placebo using a linear one-way analysis of variance (ANOVA) model in SAS® (SAS® Institute, Cary, North Carolina). Means were calculated using the LS Means method in SAS®. Standard deviations were calculated using the Statistical Summary function in SAS®. Other statistical tests were also presented in graphics. These included: linear regression of response *vs*. exposure (dose, AUC, or Cmax), Kendall-tau nonparametric regression, and Spearman nonparametric regression.

### Secondary Efficacy Variable(s)

### DLMO - Time to Onset and Lowest Effective Dose

Time (day) at which maximum advance in the circadian period occurred was determined by comparing DLMO25% from baseline and treated nights for all subjects, as described above. Additionally, the lowest effective dose was also determined by comparing DLMO25% from baseline and treated nights as described above. The first dose with a statistically significant p-value in the ANOVA with pairwise contrast was considered the lowest effective dose.

### Sleep and PSG-based Outcomes

Sleep latency (latency to persistent sleep and wake after sleep onset (WASO) were measured by PSG on Nights 1, 2, 4, 5, and 6.

The differences in these sleep parameters between the endpoint day and baseline were analyzed by comparing pairwise each dose group to placebo using a linear one-way analysis of variance (ANOVA) model in SAS® (SAS® Institute, Cary, North Carolina). Means were calculated using the LS Means method in SAS®. Standard deviations were calculated using the Statistical Summary function in SAS®. Other statistical tests were also presented in graphics. These included: linear regression of response vs. exposure (dose, AUC, or Cmax), Kendall-tau nonparametric regression, and Spearman nonparametric regression. Primary Efficacy Results

### 11.1.1.1 Shift of Dim Light Melatonin Onset

In this study, Dim Light Melatonin Onset_{25%, LOQ5} (DLMO_{25%}, _{LOQ5}) was defined as the time when melatonin production reached 25% of the maximum melatonin concentration (MELₘₐₓ) and samples below the limit of quantification (LOQ) of the melatonin assay were assigned 5 pg/ml. LOQ5 represents half of the lowest level of quantification for the assay (10 pg/ml) and is a more probable value to estimate for samples below the limit of quantification than assigning a value of zero.

MA-1, when compared to placebo, was able to induce a forward shift in DLMO_{25%}, _{LOQ5} on the first night of treatment (Night 4) when compared to baseline DLMO_{25%}, _{LOQ5} (Night 3) in a dose-dependent manner (Table 11.1.1).

**Table 11.1.1 Change in DLMO_{25%,LOQ5} between Night 4 and Night 3 by Dose***

| | **Dose Group** | | | | |
|---|---|---|---|---|---|
| DLMO_{25%, LOQ5} | Placebo | 10 mg | 20 mg | 50 mg | 100 mg |
| Change in Hours | N =6 | N =8 | N =7 | N =4 | N =5 |
| | -0.48 ± 0.84 | 0.18 | -1.14 | -0.50 | -2.74 ± 1.95 (0.0276) |
| *Values for change in DLMO (mean ± SD) are displayed for each dose group exhibiting evidence of a statistically significant effect. The p-value (in parentheses) compares that dose group to placebo using ANOVA with contrasts. | | | | | |

### Change in Sleep Efficiency

The ability of MA-1 to correct the disruption in sleep caused by a phase advance was investigated by comparing the change in sleep efficiencies of MA-1 treated subjects upon a phase advance against the change in sleep efficiencies in placebo upon a phase advance. Sleep efficiency (time asleep/opportunity to sleep * 100%) was measured objectively by overnight polysomnogramic recordings. Polysomnographic recording from baseline (Night 1 and 2) and on treatment nights 4, 5, and 6 were analyzed for this study.

### Full Night Sleep Efficiency

MA-1 was able to minimize the disruption in full night sleep efficiency between Night 4 and Night 2 in a dose-related manner. (Table 11.1.2).

**Table 11.1.2 Change in Sleep Efficiency between Night 4 and Night 2 by Dose***

| **Dose** | **Mean Change ± SD in Sleep Efficiency** | |
|---|---|---|
| | **Full Night (% points)** | **2nd Third of the Night (% points)** |
| Placebo (N=7)¹ | -20.27 ± 18.72 | -34.92 ± 38.23 |
| MA-1 10 mg (N=8)² | -7.77 | -12.64 ± 13.83 (0.0303) |
| MA-1 20 mg (N=8) | -6.68 | -5.11 ± 12.78 (0.0048) |
| MA-1 50 mg (N=7) | -5.87 ± 9.89 (0.0487) | -2.10 ± 4.14 (0.0028) |
| MA-1 100 mg (N=7) | -2.02 ± 4.94 (0.0141) | -2.30 ± 5.72 (0.0030) |
| *Values for change in sleep efficiency for the full night (mean ± SD) are displayed for each dose group exhibiting evidence of a statistically significant effect. The *p-value* (in parentheses) compares that dose group to placebo using ANOVA with contrasts. | | |

### Sleep Efficiency in Parts of the Night

Sleep efficiency was also compared in parts of the night by dividing the full night into thirds. MA-1 improved sleep efficiency in the middle third of the night in a dose-related manner. (Table 11.1.2).

### 11.1.2 Secondary Efficacy Results

### 11.1.2.1 DLMO Shift - Time to Onset and Lowest Effective Dose

As detailed in Section 11.1.1.1, MA-1, when compared to placebo, was able to induce a forward shift in DLMO_{25%}, _{LOQ5} on the first night of treatment (Night 4) when compared to baseline (Night 3) in a dose-dependent manner (Table 11.1.1, Figure 11.1.1). While nonparametric analysis clearly indicates an overall dose-response, the MA-1 100 mg dose is considered the lowest effective dose for DLMO shift since it was the first dose with a statistically significant p-value in the ANOVA with contrasts.

### 11.1.2.2 Other Sleep Parameters

In addition to sleep efficiency, the exposure-response of MA-1 on sleep latency, sleep maintenance, and sleep architecture were examined.

### Sleep Latency

MA-1, when compared to placebo, was able to reduce latency to persistent sleep (LPS) on the first night of treatment (Night 4) when compared to baseline (Night 2) (Table 11.1.3).

**Table 11.1.3 Change in Sleep Latency between Night 4 and Night 2 by dose***

| **Dose** | **Latency to Persistent Sleep (Min)** |
|---|---|
| Placebo (N=8) | 15.13 ± 21.25 |
| MA-1 10mg(N=8) | -8.25 ± 16.34 (0.0034) |
| MA-1 20 mg (N=8) | 5.00 |
| MA-150mg(N=7) | -3.71 ± 10.97 (0.0193) |
| MA-1 100mg(N=6) | -4.17 ± 6.93 (0.0214) |
| *Values for change in sleep latency (mean ± SD) are displayed for each dose group exhibiting evidence of a statistically significant effect. The P value (in parentheses) compares that dose group to placebo using ANOVA with contrasts. | |

### Sleep maintenance

**Table 11.1.4 Change in Sleep Maintenance between Night 4 and Night 2 by dose***

| **Dose** | **WASO (Min)** | **WASO (% points)** |
|---|---|---|
| Placebo (N=7) | 77.00 ± 91.01 | 17.22 ± 19.69 |
| MA-1 10 mg (N=8) | 40.56 | 8.37 |
| MA-1 20 mg (N=8) | 31.19 | 6.91 |
| MA-1 50 mg (N=7) | 31.21 | 6.61 |
| MA-1 100 mg (N=7) | 8.50 ± 20.39 (0.0452) | 1.85 ± 4.29 (0.0391) |
| *Values for change in sleep maintenance (mean ± SD) are displayed for each dose group exhibiting evidence of a statistically significant effect. The P value (in parentheses) compares that dose group to placebo using ANOVA with contrasts. | | |

Wake after sleep onset (WASO) was calculated as both a unit of time (number of minutes that a subject was awake after falling into persistent sleep) and as a fraction (fraction of time that the subject was awake in the time frame from persistent sleep to lights on). Statistical significance was achieved when the MA-1 100 mg dose was compared to placebo in WASO as both a unit of time and as a fraction (Table 11.1.4). While dose response as measured by nonparametric analyses was not statistically significant, linear regression analysis of change in WASO at each dose tested demonstrates that the MA-1 100 mg dose was able to minimize the disruption in wake after sleep onset between Day 4 and Day 2 in the majority of subjects in this treatment arm.

### Sleep Architecture and REM Polarity

MA-1 did not change the percentage of time in each sleep stage between Night 4 and Night 2.

On Night 4, MA-1 was able to minimize the disruption in REM polarity caused by a phase advance by increasing the number of episodes of REM during the final third of the night. After Hour 4 on Night 4, there were fewer cumulative episodes of REM with placebo compared to the larger doses of MA-1. This disruption in REM polarity was not observed on Night 2.

Additional analyses evaluated cumulative REM epochs during the thirds of the night. MA-1 was able to induce a dose-related increase in the number of episodes of REM during the final third of the night consistent with preserving the REM sleep architecture of Night 2 prior to the phase advance.

### Example 2.

A multi-center, randomized, double-blind, placebo-controlled, parallel-group study was conducted to investigate the efficacy and safety of single oral doses of MA-1 (20, 50, and 100 mg) and matching placebo in healthy male and female subjects with induced transient insomnia. Approximately four hundred subjects were randomized in approximately a 1:1:1:1 ratio to the treatment groups.

In general, a screening period began 14 to 35 days prior to the start of the evaluation period, which was Day 1. Prior to Day 1, subjects were asked to increase their sleep time to 9 hours per night. Drug, or placebo, was administered on Night 1, approximately 0.5 hour prior to lights off.

The primary efficacy variable was LPS. LPS is defined as the length of time elapsed between lights off and onset of persistent sleep. In this trial, persistent sleep is defined as the point at which 10 minutes of uninterrupted sleep has begun. Sleep was determined on the basis of polysomnography (PSG).

Secondary efficacy parameters included the following:
Wake After Sleep Onset (WASO): WASO is defined as the time spent awake between onset of sleep and Lights On during Night 1, determined by PSG.
Latency to Non-Awake (LNA): LNA is defined as the number of minutes to reach any stage of sleep. Total Sleep Time (TST): TST is defined as the number of minutes spent asleep during the entire time in bed.

The particle size of the MA-1 used in this study was:

| D10 | D50 | D90 | D100 |
|---|---|---|---|
| 5 um | 25 um | 72 um | 316 um |

Illustrative results included the following.
- Latency to Persistent Sleep (LPS): Improvement compared with placebo of 21.5 (p<0.001), 26.3 (p<0.001), and 22.8 (p<0.001) minutes at 20, 50, and 100 mg respectively.
- Latency to Non-Awake (LNA): Improvement compared with placebo of 11.1 (p<0.006), 14.3 (p<0.001), and 12.3 (p<0.002) minutes at 20, 50, and 100 mg respectively.
- Wake After Sleep Onset (WASO): Improvement compared with placebo of 24.2 (p<0.02), 33.7 (p=0.001), and 17.5 (p=0.081) minutes at 20, 50, and 100 mg respectively.
- Total Sleep Time (TST): Improvement compared with placebo of 33.7 (p<0.002), 47.9 (p<0.001) and 29.6 (p<0.005) minutes at 20, 50, and 100 mg respectively.

The trial also demonstrated that MA-1 was well-tolerated at all doses.

Several conclusions can be drawn from Examples 1 and 2. These include but are not necessarily limited to the following.
- MA-1 was well-tolerated at doses of 10, 20, 50, and 100mg.
- MA-1, when compared to placebo, induced a forward shift in DLMO_{25%}, _{LOQ5} on the first night of treatment in a dose-dependent manner.
- MA-1 minimized the disruption in sleep efficiency (full night and middle third of the night) caused by a phase advance.
- MA-1 minimized the disruption in REM polarity caused by a phase advance by increasing in the number of episodes of REM during the final third of the night.
- MA-1 minimized the disruption in wake after sleep onset (WASO) caused by a phase advance.
- MA-1 improved sleep latency which was increased by the phase advance.
- The Cmax values increased in a manner approximately proportional to the dose. AUC increased approximately proportional to dose.
- Exposure levels were not affected by age, weight, height, gender, creatinine clearance, or ALT baseline levels.
- 50 mg was more efficacious than 100 mg despite both doses being well-tolerated, indicating that a single oral dose of about 50 mg is preferable to an oral dose of about 100 mg.
- 20 mg was comparable or superior to 100 mg in efficacy despite 100 mg being well-tolerated, indicating that a single oral dose of about 20 mg is preferable to an oral dose of about 100 mg.
- An oral dose of about 20 to about 50 mg is effective in treating sleep disorders.
- An oral dose of about 20 to about 50 mg is effective in treating sleep disorders when administered about 1/2 hour before sleep time.

## Claims

1. Use of (1 R-trans)-N-[[2-(2,3-dihydro-4-benzofuranyl)cyclopropyl]methyl]-propanamide (MA-1) for the preparation of a pharmaceutical composition for the treatment of a circadian rhythm disorder or a sleep disorder, the pharmaceutical composition being formulated for orally administering MA-1 to the subject suffering such disorder in an amount of about 20 mg or about 50 mg per day.

2. The use of claim 1, wherein the pharmaceutical composition is intended for administration at or within about 2 hours prior to bedtime.

3. The use of claim 2, wherein the pharmaceutical composition is intended for administration about 1/2 hour before sleep time.

## Patentansprüche

1. Verwendung von (1R-trans)-N-[[2-(2,3-Dihydro-4-benzofuranyl)cyclopropyl]methyl]propanamid (MA-1) für die Herstellung einer pharmazeutischen Zusammensetzung für die Behandlung einer Störung des Biorhythmus oder einer Schlafstörung, wobei die pharmazeutische Zusammensetzung zur oralen Verabreichung von MA-1 an die Person, die unter einer solchen Störung leidet, in einer Menge von etwa 20 mg bis etwa 50 mg pro Tag formuliert ist.

2. Verwendung nach Anspruch 1, wobei die pharmazeutische Zusammensetzung zur Verabreichung zur oder innerhalb von 2 Stunden vor der Bettgehzeit Gedacht ist.

3. Verwendung nach Anspruch 2, wobei die pharmazeutische Zusammensetzung zur Verabreichung ungefähr ½ Stunde vor der Schlafenszeit gedacht ist.

## Revendications

1. Utilisation de (1 R-trans)-N-[[2-(2,3-dihydro-4-benzofuranyle)cyclopropyle]méthyle]-propanamide (MA-1) pour la préparation d'une composition pharmaceutique pour le traitement d'un trouble du rythme circadien ou d'un trouble du sommeil, la composition pharmaceutique étant formulée pour administration par voie orale du MA-1 au sujet qui souffre dudit trouble en une quantité d'environ 20 mg à environ 50 mg par jour.

2. Utilisation selon la revendication 1, dans laquelle la composition pharmaceutique est prévue pour être administrée au coucher ou durant les 2 heures environ précédant le coucher.

3. Utilisation selon la revendication 2, dans laquelle la composition pharmaceutique est prévue pour être administrée environ une demi-heure avant l'endormissement.
